# EUROPEAN PATENT SPECIFICATION

(11) **EP 2 345 410 B1**
(45) Date of publication and mention of the grant of the patent: **30.12.2020**
(21) Application number: 09817723.1
(22) Date of filing: 28.09.2009
(51) Int. Cl.: A61K 31/426, A61K 47/02, A61K 47/10, A61K 47/12, A61K 47/14, A61K 47/18, A61K 47/22, A61K 47/26, A61K 47/32, A61K 47/34, A61K 47/38, A61P 3/10, A61P 13/10

(54) **PHARMACEUTICAL COMPOSITION FOR MODIFIED RELEASE**
PHARMAZEUTISCHE ZUSAMMENSETZUNG ZUR MODIFIZIERTEN FREISETZUNG
COMPOSITION PHARMACEUTIQUE À LIBÉRATION MODIFIÉE

(30) Priority: 30.09.2008 US 101338 P
(43) Date of publication of application: 20.07.2011
(73) Proprietor: Astellas Pharma Inc., Chuo-ku Tokyo 103-8411 (JP)
(72) Inventor: TAKAISHI Yuuki, Tokyo 103-8411 (JP); TAKAHASHI Yutaka, Tokyo 103-8411 (JP); NISHIZATO Takashi, Tokyo 103-8411 (JP); MURAYAMA Daisuke, Tokyo 103-8411 (JP); MURAYAMA Emiko, Tokyo 103-8411 (JP); NAKAMURA Soichiro, Tokyo 103-8411 (JP); SAKO Kazuhiro, Tokyo 103-8411 (JP)
(74) Representative: Hoffmann Eitle
(86) International application number: PCT/JP2009/066742
(87) International publication number: WO 2010/038690

(56) References cited:
- EP-A1- 1 028 111
- EP-A1- 1 440 969
- WO-A1-94/06414
- WO-A1-2004/041276
- WO-A1-2008/084698
- JP-A- 2005 162 737
- JP-T- 2005 519 884

## Description

### TECHNICAL FIELD

The present invention relates to a pharmaceutical composition for modified release capable of reducing food effects, which are observed in conventional tablets, by combining an active ingredient with specific ingredients to control a releasing rate of the active ingredient.

More particularly, the present invention relates to a pharmaceutical composition comprising (R)-2-(2-aminothiazol-4-yl)-4'-[2-[(2-hydroxy-2-phenylethyl)amino]ethyl]acetic acid anilide or a pharmaceutically acceptable salt thereof, an additive which ensures penetration of water into the pharmaceutical composition (hereinafter sometimes referred to as a hydrophilic base), and a polymer which forms a hydrogel, in which the changes in AUC and Cmax caused by the intake of food can be decreased by controlling a releasing rate of the active ingredient.

### BACKGROUND ART

(R)-2-(2-aminothiazol-4-yl)-4'-[2-[(2-hydroxy-2-phenylethyl)amino]ethyl]acetic acid anilide has been created by Astellas Pharma Inc., and it has been reported that this compound has not only both an activity of promoting insulin secretion and an activity of enhancing insulin sensitivity, but also an antiobestic activity and an antihyperlipemic activity based on an activity of selectively stimulating a β3 receptor, and is useful in treating diabetes (see, for example WO 99/20607; Example 41) .

Further, it has been reported e.g. in WO 2004/041276 that the compound can be used as a therapeutic agent for overactive bladder, such as overactive bladder accompanied by prostatic hyperplasia, or overactive bladder accompanied by urinary urgency, urinary incontinence, and urinary frequency. WO 2004/041276 also describes a tablet formulation of (R)-2-(2-aminothiazol-4-yl)-4'-[2-[(2-hydroxy-2-phenylethyl)amino]ethyl]acetic acid anilide.

A clinical trial of (R)-2-(2-aminothiazol-4-yl)-4'-[2-[(2-hydroxy-2-phenylethyl)amino]ethyl]acetic acid anilide in the form of conventional formulations revealed disadvantages, for example, that pharmacokinetic data unexpectedly varied according to the presence or absence of the intake of food (not published). For example, the rate of decrease of Cmax in a fed state was 67%, and the rate of decrease of AUC in the fed state was 47%, in comparison with those in a fasted state. In this case, Cmax in the fasted state was three times higher than that in the fed state. These problems are considered to be raised by, for example, the changes in pharmacokinetics caused by food, and therefore, the development of a formulation capable of avoiding the effects by food intake is desired.

As a technique of preparing a formulation for modified release, a hydrogel sustained release tablet containing an additive which ensures penetration of water into the tablet, and a hydrogel-forming polymer is disclosed (see, for example, WO 94/06414).

However, WO 94/06414 does not refer to (R)-2-(2-aminothiazol-4-yl)-4'-[2-[(2-hydroxy-2-phenylethyl)amino]ethyl]acetic acid anilide, and further improvements are needed to produce a pharmaceutical composition.

### SUMMARY OF INVENTION

### TECHNICAL PROBLEM

An object of the present invention is to provide a pharmaceutical composition for modified release comprising (R)-2-(2-aminothiazol-4-yl)-4'-[2-[(2-hydroxy-2-phenylethyl)amino]ethyl]acetic acid anilide or a pharmaceutically acceptable salt thereof, in which the pharmaceutical composition has efficacy the same as or higher than those of conventional formulations and has no limitations on food intake, and a process of manufacturing the pharmaceutical composition.

### SOLUTION TO PROBLEM

The elimination half-life (T_{1/2}) of (R)-2-(2-aminothiazol-4-yl)-4'-[2-[(2-hydroxy-2-phenylethyl)amino]ethyl]acetic acid anilide is long (approximately 18 to 24 hours), and thus, a formulation thereof for modified release is not necessarily needed to maintain its blood level. Taking into consideration the results of the clinical trial described above, the present inventors conducted intensive studies to design the formulation by paying attention to the control of a release rate of the drug from a formulation to the extent that the release is not affected by food intake or the like, rather than the addition of release control.

On the basis of blood concentration profiles (in a fasted state/after the intake of food) after administration of a conventional formulation (rapid release formulation), the absorption rate of the drug in a fed state was calculated by a deconvolution method to predict continuous absorption for about 4 hours. The present inventors considered from this result that a formulation capable of continuous drug release for 4 hours or more would be able to reduce the effects by food, because the drug release from the formulation would become the rate-limiting step for absorption.

The present inventors carried out a clinical trial in human using three types of formulations in which the release rate of the drug was controlled (Time when the release percentage of the drug from the unit formulation was 80% (T80%) = 4 hr, 6 hr, and 10 hr), and found that all formulations could reduce the effects by food, to complete the present invention.

It is generally known that the retention time in the stomach and the release rate of formulations for modified release vary according to the presence or absence of food intake, and as a result, there is a possibility that blood concentration profiles is changed. However, surprisingly, when using this formulation, the change of the blood concentration profiles was small in the presence or absence of food intake.

The present invention is characterized by providing a pharmaceutical composition for modified release which is not affected by the effects of food intake and exhibits a decreased change in AUC or Cmax.

The present invention provides:
[1] A pharmaceutical composition for modified release, comprising (1) (R)-2-(2-aminothiazol-4-yl)-4'-[2-[(2-hydroxy-2-phenylethyl)amino]ethyl]acetic acid anilide, or a pharmaceutically acceptable salt thereof, (2) at least one additive which ensures penetration of water into the pharmaceutical composition and which has a solubility such that the volume of water required for dissolving 1 g of the additive is 10 mL or less, and (3) a hydrogel-forming polymer having an average molecular weight of 100,000 to 5,000,000 or a viscosity of 12 mPa·s or more in a 5% aqueous solution at 25°C and 7,500 mPa·s or less in a 1% aqueous solution at 25°C,
   wherein the additive which ensures penetration of water into the pharmaceutical composition is one compound, or two or more compounds selected from the group consisting of polyethylene glycol, polyvinylpyrrolidone, D-mannitol, lactose, sucrose, sodium chloride, and polyoxyethylene polyoxypropylene glycol;
   wherein the hydrogel-forming polymer is one compound, or two or more compounds selected from the group consisting of polyethylene oxide, hydroxypropyl methylcellulose, and hydroxypropyl cellulose; and
   wherein the drug dissolution rate from the pharmaceutical composition is 75% or less after 1.5 hours and at least 75% after 7 hours from the beginning of the dissolution test and wherein the dissolution test is carried out in accordance with the paddle method described in the United States Pharmacopoeia under the conditions that 900 mL of USP buffer, pH 6.8, is used and the paddle rotation speed is 50 to 200 rpm.
[2] The pharmaceutical composition for modified release according to [1], wherein the paddle rotation speed is 200 rpm.
[3] The pharmaceutical composition for modified release according to [1], wherein the hydrogel-forming polymer is polyethylene oxide.
[4] The pharmaceutical composition for modified release according to any one of [1] to [3], wherein an amount of the additive which ensures penetration of water into the pharmaceutical composition is 5% by weight to 75% by weight with respect to the total weight of the pharmaceutical composition.
[5] The pharmaceutical composition for modified release according to [4], wherein an amount of the additive which ensures penetration of water into the pharmaceutical composition is 5% by weight to 70% by weight with respect to the total weight of the pharmaceutical composition.
[6] The pharmaceutical composition for modified release according to any one of [1] to [5], wherein an amount of the hydrogel-forming polymer is 1% by weight to 70% by weight with respect to the total weight of the pharmaceutical composition.
[7] The pharmaceutical composition for modified release according to any one of [1] to [6], further comprising an antioxidant.
[8] The pharmaceutical composition for modified release according to [7], wherein the antioxidant is one compound, or two or more compounds selected from the group consisting of butyl hydroxytoluene, propyl gallate, and sodium ascorbate.
[9] The pharmaceutical composition for modified release according to [8], wherein the antioxidant is butyl hydroxytoluene.
[10] The pharmaceutical composition for modified release according to any one of [7] to [9], wherein an amount of the antioxidant is 0.025% by weight to 0.25% by weight.
[11] The pharmaceutical composition for modified release according to any one of [1] to [10], further comprising a stabilizer.
[12] The pharmaceutical composition for modified release according to [11], wherein the stabilizer is one compound, or two or more compounds selected from the group consisting of yellow ferric oxide, red ferric oxide, and black iron oxide.
[13] The pharmaceutical composition for modified release according to [12], wherein the stabilizer is yellow ferric oxide and/or red ferric oxide.
[14] The pharmaceutical composition for modified release according to any one of [11] to [14], wherein an amount of the stabilizer is 0.05% by weight to 1% by weight.

As formulation techniques for reducing or avoiding the changes in pharmacokinetics such as AUC or Cmax accompanied by food intake, a formulation technique concerning a sustained-release pharmaceutical composition containing tamsulosin hydrochloride is disclosed (see Japanese Unexamined Patent Publication (Kokai) No. 2005-162736 and Japanese Unexamined Patent Publication (Kokai) No. 2005-162737). This formulation technique is limited to tamsulosin, and applied to a formulation containing the drug at a low dose (0.4 mg per unit formulation). This formulation enables to control the release of tamsulosin therefrom by being mainly composed of a sustained-release base. By contrast, the pharmaceutical composition contains the drug at a high dose (i.e., high content per unit formulation), and it is considered difficult to control the release rate of the drug from a formulation containing the sustained-release base at a low content, and therefore, the present invention is technically quite different from the formulation disclosed in these references.

### ADVANTAGEOUS EFFECTS OF INVENTION

According to the present invention, a pharmaceutical composition for modified release which has no limitations on food intake and is stable (for example, reduction of changes in a sequential dissolution profile) can be provided.

Further, a pharmaceutical composition for modified release in which AUC is not reduced can be provided.

With respect to a conventional formulation, the rate of decrease of Cmax in the fed state was 67% in comparison with that in a fasted state. By contrast, with respect to the pharmaceutical composition for modified release of the present invention, the rate of decrease of Cmax in the fed state was 42% in comparison with that in a fasted state, and this result showed that reduction of Cmax caused by food intake could be significantly alleviated by forming its formulation into the pharmaceutical formulation for modified release.

### BRIEF DESCRIPTION OF DRAWINGS

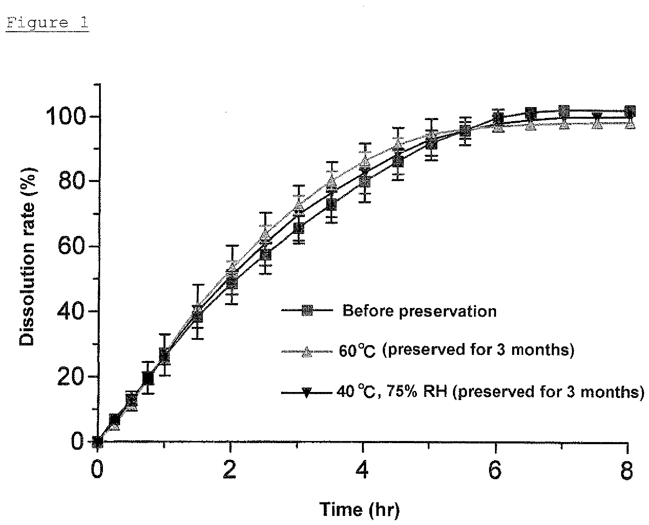

[Fig. 1] Figure 1 is a graph showing dissolution profiles of the pharmaceutical composition for modified release prepared in Example 11, and the time courses thereof.

### DESCRIPTION OF EMBODIMENTS

The pharmaceutical composition for modified release of the present invention will be explained hereinafter.

The term "rapid release formulation (conventional formulation)" as used herein means a formulation in which the dissolution rate of the drug from the formulation is 85% or more after 30 minutes from the beginning a dissolution test, which is carried out in accordance with a dissolution test (paddle method) described in the United States Pharmacopoeia under the conditions that 900 mL of an appropriate test fluid (such as a USP buffer, pH 6.8) is used and the paddle rotation speed is 100 rpm. Alternatively, the term means a formulation in which the dissolution rate of the drug from the formulation is 85% or more after 30 minutes from the beginning a dissolution test, which is carried out in accordance with a dissolution test, method 2 described in the Japanese Pharmacopoeia under the conditions that 900 mL of an appropriate test fluid (such as a Mc. Ilvain buffer, pH 6.8) is used and the paddle rotation speed is 50 rpm.

The term "pharmaceutical composition for modified release" as used herein means a formulation in which the dissolution rate of the drug from the formulation is less than 85% after 30 minutes from the beginning a dissolution test carried out under the above conditions, and the drug release is controlled to the extent that the effects by food are reduced. More particularly, it is a formulation in which an additive (hydrophilic base) which ensures penetration of water into the formulation is combined with a polymer which forms a hydrogel.

The wording "the effects by food are reduced" as used herein means, for example, a 10% reduction, a 20% reduction in another embodiment, and a 30% reduction in still another embodiment, in comparison with Cmax of a conventional formulation. Alternatively, the term means, for example, a 10% reduction with respect to the rates of decrease of Cmax and AUC in administration after food intake, in comparison with Cmax and AUC in administration in the fasted state, a 20% reduction in another embodiment, and a 30% reduction in still another embodiment.

The rates of decrease of Cmax and AUC are calculated by the following equations: $Rd \left(Cmax\right) = \left[Cmax\left(FS\right)-Cmax\left(FI\right)\right] \times 100 / Cmax \left(FS\right)$ $Rd \left(AUC\right) = \left[AUC\left(FS\right)-AUC\left(FI\right)\right] \times 100 / AUC \left(FS\right)$
Rd(Cmax): Rate of decrease of Cmax (%)
Cmax(FS): Cmax in administration in the fasted state
Cmax(FI): Cmax in administration after food intake
Rd(AUC): Rate of decrease of AUC (%)
AUC(FS): AUC in administration in the fasted state
AUC(FI): AUC in administration after food intake

The term "formulation in which the effects by food are reduced" as used herein means a formulation in which the dissolution rate of the drug from the formulation is 75% or less after 1.5 hours and 75% or more to 100% or less after 7 hours from the beginning a dissolution test, which is carried out in accordance with a dissolution test (paddle method) described in the United States Pharmacopoeia under the conditions that 900 mL of USP buffer, pH 6.8 is used and the paddle rotation speed is 50 to 200 rpm.

The term "stable" as used herein means that it is stable against, for example, heat, temperature, humidity, or light. More particularly, the term means that, for example, when a plastic bottle is filled with a pharmaceutical composition and sealed, and then, the bottle is preserved for three months under the conditions at 40°C and 75% RH or at 60°C, the change in the dissolution rate at the point showing a dissolution rate of 50% is within ±5% or less. Alternatively, the term means that, for example, when a pharmaceutical composition is exposed to 1.2 million Lux·hr of light, the change in the dissolution rate at the point showing a dissolution rate of 50% is within ±5% or less.

(R)-2-(2-aminothiazol-4-yl)-4'-[2-[(2-hydroxy-2-phenylethyl)amino]ethyl]acetic acid anilide (hereinafter sometimes referred to as compound A) is represented by the following structural formula.

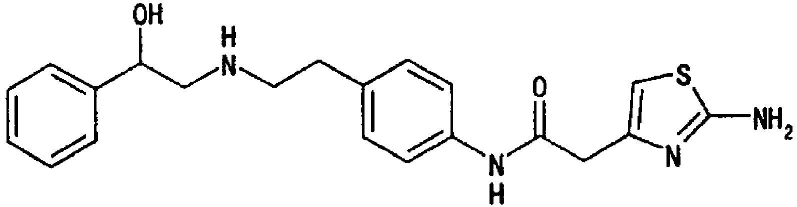

Compound A may be used in a free form which is not a salt, and may form a salt with an acid in other embodiments. Examples of such a salt include an acid addition salt with a mineral acid such as hydrochloric acid, hydrobromic acid, hydroiodic acid, sulfuric acid, nitric acid, phosphoric acid, or the like; and an acid addition salt with an organic acid such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, ethanesulfonic acid, glutamic acid, or the like.

The dose of compound A may be appropriately selected in accordance with symptom, age, sex, and the like of the patient to be treated. The daily dose of compound A for oral administration to an adult is generally 0.01 to 100 mg/kg, which is administered once or divided into two to four doses per day.

The content of compound A per formulation is, for example, 1% by weight to 70% by weight, 5% by weight to 70% by weight in another embodiment, and 5% by weight to 50% by weight in still another embodiment. The content of compound A per formulation is 1 mg to 500 mg, and 10 mg to 200mg in another embodiment.

It is necessary that the hydrogel-forming polymer used in the present invention can control the release rate of the drug, to the extent that the blood concentration profile of the drug is not affected by the presence or absence of food intake.

The molecular weight of the hydrogel-forming polymer is 100,000 to 5,000,000 preferably 100,000 to 2,000,000. The viscosity of the hydrogel-forming polymer is 12 mPa·s or more in a 5% aqueous solution at 25°C, and 7,500 mPa·s or less in a 1% aqueous solution at 25°C, preferably 5,500 mPa· s or less in a 1% aqueous solution at 25°C.

In the pharmaceutical composition for modified release of the present invention, the release period of time of the drug from the formulation can be arbitrarily controlled by adjusting the viscosity of the polymer which is used as the hydrogel-forming polymer.

The hydrogel-forming polymer controls release of the drug to the extend that the effects of food on compound A may be reduced. The hydrogel-forming polymer is polyethylene oxide, hydroxypropyl methylcellulose, and hydroxypropyl cellulose.

Examples of polyethylene oxide (hereinafter sometimes referred to as PEO) include product names, Polyox WSR Coagulant [average molecular weight: 5,000,000, viscosity: 5,500-7,500 mPa·s (1% aqueous solution at 25°C)], Polyox WSR-301 [average molecular weight: 4,000,000, viscosity: 1,650-5,500 mPa·s (1% aqueous solution at 25°C)], Polyox WSR-N-60K [average molecular weight: 2,000,000, viscosity: 2,000-4,000 mPa·s (2% aqueous solution at 25°C)], Polyox WSR-N-12K [average molecular weight: 1,000,000, viscosity: 400-800 mPa·s (2% aqueous solution at 25°C)], Polyox WSR-1105 [average molecular weight: 900,000, viscosity: 8,800-17,600 mPa·s (5% aqueous solution at 25°C)], Polyox WSR-205 [average molecular weight: 600,000, viscosity: 4,500-8,800 mPa·s (5% aqueous solution at 25°C)], Polyox WSR-N-750 [average molecular weight: 300,000, viscosity: 600-1200 mPa· s (5% aqueous solution at 25°C)], Polyox WSR-N-80 [average molecular weight: 200,000, viscosity: 55-90 mPa·s (5% aqueous solution at 25°C)], and Polyox WSR-N-10 [average molecular weight: 100,000, viscosity: 12-50 mPa·s (5% aqueous solution at 25°C)](DOW).

Examples of hydoxypropyl methylcellulose (hereinafter sometimes referred to as HPMC) include product name Metolose 90SH50000 [viscosity in a 2% aqueous solution at 20°C: 2,900-3,900 mPa·s], Metolose SB-4 (product name, Shin-Etsu Chemical Co., Ltd.)(viscosity in a 2% aqueous solution at 20°C: approximately 4mPa·S), TC-5RW (product name, Shin-Etsu Chemical Co., Ltd.)(viscosity in a 2% aqueous solution at 20°C: approximately 6mPa·S), TC-5S (product name, Shin-Etsu Chemical Co., Ltd.)(viscosity in a 2% aqueous solution at 20°C: approximately 15mPa·S), TC-5R (product name, Shin-Etsu Chemical Co., Ltd.)(viscosity in a 2% aqueous solution at 20°C: approximately 6mPa·S), TC-5M (product name, Shin-Etsu Chemical Co., Ltd.)(viscosity in a 2% aqueous solution at 20°C: approximately 4.5mPa·S), TC-5E (product name, Shin-Etsu Chemical Co., Ltd.)(viscosity in a 2% aqueous solution at 20°C: approximately 3mPa·S), Metolose 60SH-50 (product name, Shin-Etsu Chemical Co., Ltd.)(viscosity in a 2% aqueous solution at 20°C: approximately 50 mPa·s), Metolose 65SH-50 (product name, Shin-Etsu Chemical Co., Ltd.)(viscosity in a 2% aqueous solution at 20°C: approximately 50 mPa·s), Metolose 90SH-100 (product name, Shin-Etsu Chemical Co., Ltd.)(viscosity in a 2% aqueous solution at 20°C: approximately 100 mPa·s), Metolose 90SH-100SR (product name, Shin-Etsu Chemical Co., Ltd.)(viscosity in a 2% aqueous solution at 20°C: approximately 100 mPa·s), Metolose 65SH-400 (product name, Shin-Etsu Chemical Co., Ltd.)(viscosity in a 2% aqueous solution at 20°C: approximately 400 mPa·s), Metolose 90SH-400 (product name, Shin-Etsu Chemical Co., Ltd.)(viscosity in a 2% aqueous solution at 20°C: approximately 400 mPa·s), Metolose 65SH-1500 (product name, Shin-Etsu Chemical Co., Ltd.)(viscosity in a 2% aqueous solution at 20°C: approximately 1,500 mPa·s), Metolose 60SH-4000 (product name, Shin-Etsu Chemical Co., Ltd.)(viscosity in a 2% aqueous solution at 20°C: approximately 4,000 mPa·s), Metolose 65SH-4000 (product name, Shin-Etsu Chemical Co., Ltd.)(viscosity in a 2% aqueous solution at 20°C: approximately 4,000 mPa·s), Metolose 90SH-4000 (product name, Shin-Etsu Chemical Co., Ltd.)(viscosity in a 2% aqueous solution at 20°C: approximately 4,000 mPa·s), Metolose 90SH-4000SR (product name, Shin-Etsu Chemical Co., Ltd.)(viscosity in a 2% aqueous solution at 20°C: approximately 4,000 mPa·s), Metolose 90SH-15000 (product name, Shin-Etsu Chemical Co., Ltd.)(viscosity in a 2% aqueous solution at 20°C: approximately 15,000 mPa·s), Metolose 90SH-15000SR (product name, Shin-Etsu Chemical Co., Ltd.)(viscosity in a 2% aqueous solution at 20°C: approximately 15,000 mPa·s), and Metolose 90SH-30000 (product name, Shin-Etsu Chemical Co., Ltd.)(viscosity in a 2% aqueous solution at 20°C: approximately 30,000 mPa·s).

Examples of hydroxypropyl cellulose (hereinafter sometimes referred to as HPC) include HPC-SSL (product name, Nippon Soda Co., Ltd.)(viscosity in a 2% aqueous solution at 20°C: 2.0-2.9 mPa·S), HPC-SL (product name, Nippon Soda Co., Ltd.)(viscosity in a 2% aqueous solution at 20°C: 3.0-5.9 mPa·S), HPC-L (product name, Nippon Soda Co., Ltd.)(viscosity in a 2% aqueous solution at 20°C: 6.0-10.0 mPa·S), HPC-M (product name, Nippon Soda Co., Ltd.)(viscosity in a 2% aqueous solution at 20°C: 150-400 mPa·S), and HPC-H (product name, Nippon Soda Co., Ltd.)(viscosity in a 2% aqueous solution at 20°C: 1,000-4,000 mPa·S).

These hydrogel-forming polymers may be used alone, or as an appropriate combination of two or more thereof. A combination of different lots may be used.

The content of the hydrogel-forming polymer is not particularly limited, so long as it is an amount to the extent that the blood concentration profile of the drug is not affected by the presence or absence of food intake. The content of the hydrogel-forming polymer is, for example, 1% by weight to 70% by weight with respect to the total weight of the formulation, and 3% by weight to 70% by weight in another embodiment. The content of the hydrogel-forming polymer is 5% by weight to 70% by weight with respect to the total weight of the formulation, 10% by weight to 60% by weight in another embodiment, and 10% by weight to 40% by weight in still another embodiment. The content of the hydrogel-forming polymer is 0.1% by weight to 1,000% by weight with respect to the weight of the drug, 1% by weight to 500% by weight in another embodiment, and 5% by weight to 300% by weight in still another embodiment.

A polymer of which the viscosity (before mixing) is beyond the specific range can be used as an appropriate combination with one or more other polymers, in case that the mixture obtained by mixing these plural polymers has a viscosity (as measured before the use) within the specific range.

In the additive which ensures penetration of water into the pharmaceutical composition of the present invention (hydrophilic base), the amount of water necessary to dissolve 1 g of the hydrophilic base at 20±5°C is 10 mL or less, 6 mL or less in another embodiment, 5 mL or less in still another embodiment, and 4 mL or less in still another embodiment. When the hydrophilic base has a high solubility to water, the effect that allows water to penetrate into the formulation is high.

The hydrophilic base is polyethylene glycol [PEG: for example, product names PEG 400, PEG 1500, PEG 4000, PEG 6000, and PEG 20000 (NOF Corporation)], polyvinyl pyrrolidone (PVP: for example, product name PVP K30 (BASF), D-mannitol, lactose, sucrose, polyoxyethylene polyoxypropylene glycol [for example, Pluronic F68 (Asahi Denka and the like)] or sodium chloride.

These hydrophilic bases may be used alone, or as an appropriate combination of two or more thereof.

The content of the hydrophilic base is not particularly limited, so long as it is an amount capable of controlling the release of the drug to the extent that the release of the drug is not affected by food. The content of the hydrophilic base is, for example, 5% by weight to 75% by weight, 5% by weight to 70% by weight in another embodiment, and 20% by weight to 60% by weight in still another embodiment.

The pharmaceutical composition for modified release of the present invention may be prepared as various dosage forms, which include, for example, formulations for oral administration such as tablets, capsules (including microcapsules), granules, and powder, and formulations for parenteral administration such as suppositories (for example, rectal suppositories or vaginal suppositories). These formulations may be safely administered orally or parenterally. Formulations for oral administration such as tablets, capsules, and granules may be selected in another embodiment.

The pharmaceutical composition for modified release of the present invention may be prepared by mixing the drug, the hydrogel-forming polymers, and the hydrophilic base, and forming the mixture into a predetermined shape. The mixing and forming may be carried out in accordance with conventional methods widely used in the technical field for formulation. A pharmaceutically acceptable carrier may be used in the mixing and/or forming, if desired.

In the preparation of the pharmaceutical composition for modified release of the present invention, further various pharmaceutical additives may be used, if desired. Such pharmaceutical additives are not particularly limited, so long as they are pharmaceutically acceptable. Examples of the pharmaceutical additives include various organic or inorganic carrier substances which are widely used as formulation materials, such as fillers, lubricants, binders, and disintegrating agents. Other formulation additives such as preservatives, antioxidants, stabilizers, film coating agents, coloring agents, and sweeteners may be used, if desired.

Examples of the fillers include lactose, sucrose, D-mannitol, D-sorbitol, starch, gelatinized starch, dextrin, crystalline cellulose, low substituted hydroxypropyl cellulose, carboxymethyl cellulose sodium, gum arabic, dextrin, pullulan, light anhydrous silicic acid, synthetic aluminum silicate, magnesium aluminate metasilicate, and the like.

Examples of the lubricants include magnesium stearate, calcium stearate, talc, colloidal silica, and the like.

Examples of the binders include gelatinized starch, sucrose, gelatin, gum arabic, methylcellulose, carboxymethyl cellulose, carboxymethyl cellulose sodium, crystalline cellulose, sucrose, D-mannitol, trehalose, dextrin, pullulan, hydroxypropyl cellulose, hydroxypropyl methylcellulose, polyvinylpyrrolidone, and the like.

Examples of the disintegrating agents include lactose, sucrose, starch, carboxymethyl cellulose, carboxymethyl cellulose calcium, croscarmellose sodium, carboxymethyl starch sodium, light anhydrous silicic acid, low substituted hydroxypropylcellulose, and the like.

Examples of the preservatives include p-hydroxybenzoate esters, chlorobutanol, benzyl alcohol, phenethyl alcohol, dehydroacetic acid, sorbic acid, and the like.

The antioxidants are not particularly limited, so long as it can avoid the effects of dissolution behavior. Examples of the antioxidants include butylated hydroxytoluene (BHT), propyl gallate (PG), butylhydroxyanisol (BHA), ascorbic acid, sodium ascorbate, erythorbic acid, sodium nitrite, sodium bisulfite, sodium pyrosulfite, citric acid, and edetate sodium; BHT, PG, and sodium ascorbate in another embodiment; and BHT in still another embodiment.

Examples of the stabilizers include yellow ferric oxide, red ferric oxide, black iron oxide, and the like.

Examples of the film coating agents include pharmaceutically commonly-used bases, such as water-soluble polymers, plasticizers, and inorganic substances, or a combination thereof.

Examples of the coloring agents include water-soluble edible tar pigments (examples: edible pigments such as food red No. 2, food red No. 3, food yellow No. 4, food yellow No. 5, food blue No. 1, and food blue No. 2), water-insoluble lake pigments (examples: aluminum salts of the above water-soluble edible tar pigments), natural pigments (examples: β-carotene, chlorophyll, and colcothar), and the like.

Examples of the sweeteners include saccharin sodium, dipotassium glycyrrhizinate, aspartame, stevia, and the like.

These carriers or formulation additives may be used alone, or as an appropriate combination of two or more thereof.

With respect to the contents thereof, they may be used in appropriate amounts. For example, the content of the antioxidant is 0.025% by weight to 0.25% by weight with respect to the total weight of the formulation, and that of the stabilizer is 0.05% by weight to 1% by weight with respect to the total weight of the formulation.

Hereinafter, the process of manufacturing the pharmaceutical composition for modified release of the present invention will be explained, the present invention is not limited to the following particular embodiments.

The pharmaceutical composition for modified release of the present invention may be prepared by known methods per se, such as dry granulation, wet granulation, fluidized bed granulation, intermittent granulation, agitation granulation, or the like.

As a method of de-lumping or pulverizing the drug, conventional crushing or pulverizing methods may be applied, for example, using an impact mill (Hosokawa Micron Corporation; Fine Impact Mill), a dry & wet mill (Powrex Corporation: Comil), or a cutting mill granulator (Dalton Corporation; Power Mill).

As a method of pulverizing the hydrophilic base, the hydrogel-forming polymer, or the formulation additives, conventional pulverizing methods may be applied, for example, using an impact mill (Hosokawa Micron Corporation; Fine Impact Mill or Sample Mill) or a jet mill (Horkos Corp; Jet Mill).

As a method of granulating the drug, conventional granulation methods may be used. Examples of such methods include a fluidized bed granulation method, an intermittent granulation method, an agitation granulation method, a high-speed agitation granulation method, a tumbling fluidized bed granulation method, an extrusion granulation method, a pulverization granulation method, a dry granulation method, and the like. In another embodiment, examples thereof include a fluidized bed granulation method, an intermittent granulation method, an agitation granulation method, a high-speed agitation granulation method, a tumbling fluidized bed granulation method, and a dry granulation method, and any method capable of granulating the drug may be used. Examples of a granulator include a fluidized bed granulator (for example, Flow Coater; Freund Corporation, or GPCG; Glatt GmbH), a granulation and coating apparatus equipped with a horizontal rotating disc having a flat powder contact portion [for example, a centrifugal fluidizing granulator (for example, CF granulator; Freund Corporation)], a granulation and coating apparatus having a rotating disk with a flat surface placed at the bottom of a fluidized bed and having an aeration portion (for example, Spiralflow, or Flowcoater with a rotor container; Freund Corporation), and a dry granulator in which material powder is directly compressed, molded, crushed, and sieved (for example, Roller Compactor; Freund Corporation).

In the dry granulation, for example, the drug, the hydrogel-forming polymer, the hydrophilic base, and additives such as a filler may be compression-molded using a dry granulator, and then, may be crushed and sieved to obtain granulated products having a desired size.

In the wet granulation, for example, while the drug, the hydrogel-forming polymer, the hydrophilic base, and additives such as a filler is fluidized, an appropriate amount of water or a liquid containing the hydrophilic base and the binder may be sprayed. The liquid containing the hydrophilic base may be prepared by dissolving or dispersing the essential component in a solvent such as water, ethanol, methanol, or the like. These solvents may be used as an appropriate mixture thereof.

The amount of water used in the granulation is not particularly limited, so long as the binder or formulation additives may be uniformly dissolved and/or suspended (dispersed) in the water. When the hydrophilic base is used in the solid form, the amount of water is not particularly limited, so long as the hydrogel-forming polymer can be granulated.

When the hydrophilic base is used in the liquid form, the amount of water to the hydrogel-forming polymer is generally 10% by weight or less, 8% by weight or less in another embodiment, and 5% by weight or less in still another embodiment. A method of adding water in the granulation is not particularly limited, so long as a nonuniform mixture consisting of untreated powder and aggregates, which are generally powdery, is not generated. Examples thereof include a continuous spray method in which water is continuously added, an intermittent spray method in which a dry step (and a shaking step, if desired) is carried out during the granulation step, and the like.

The addition rate of water in the granulation is not particularly limited, so long as a nonuniform mixture consisting of untreated powder and aggregates, which are generally powdery, is not generated. In the fluidized bed granulation, the addition rate of water to the hydrogel-forming polymer is generally 0.1% by weight/min. to 1% by weight/min., 0.2% by weight/min. to 0.8% by weight/min. in another embodiment, and 0.4% by weight/min. to 0.6% by weight/min. in still another embodiment.

The temperature of the powder in the granulation is not particularly limited, so long as it does not induce thermal denaturation of the hydrogel-forming polymer. The temperature is, for example, 20°C to the melting point (62°C to 67°C) of the hydrogel-forming polymer, 20°C to 50°C in another embodiment, 20°C to 35°C in still another embodiment, and 25°C to 30°C in still another embodiment.

The concentration of the binder liquid as a solid content which may be used in the granulation is, for example, 1% to 20% as a formulation amount. The binder is not particularly limited, so long as it is pharmaceutically acceptable.

The binder may be added in the solid form to a granulator, and then, water may be sprayed as the binder liquid. Alternatively, the binder may be dissolved in water, and then, the resulting binder liquid may be sprayed.

An appropriate spray rate of the binder liquid varies according to a production method to be applied or its production scale. In a 1-kg scale production by the fluidized bed granulation, the spray rate is 2 g/min. to 20 g/min., and 5 g/min. to 15 g/min. in another embodiment.

An appropriate temperature of the product in the granulation is 15°C to 50°C, and 15°C to 40°C in another embodiment.

The resulting granulated products may be, for example, dried or heated.

In the drying step, an apparatus and a method are not particularly limited, so long as the granulated products can be dried. Examples of an apparatus for drying include a fluidized bed granulator (for example, Flow Coater; Freund Corporation, or GPCG; Glatt GmbH), a granulation and coating apparatus equipped with a horizontal rotating disc having a flat powder contact portion [for example, a centrifugal fluidizing granulator (for example, CF granulator; Freund Corporation)], a granulation and coating apparatus having a rotating disk with a flat surface placed at the bottom of a fluidized bed and having an aeration portion (for example, Spiralflow, or Flowcoater with a rotor container; Freund Corporation), and the like. The conditions for drying are not particularly limited, so long as the granulated products may be generally dried in the fluidized bed. The drying of the granulated products will be almost completed, for example, under the conditions in which the dry inlet air temperature is 50°C and the drying is carried out until the temperature of the granulated products becomes 40°C and, in another embodiment, under the conditions in which the dry inlet air temperature is 40°C and the drying is carried out until the temperature of the granulated products becomes 30°C. As the drying method, forced-air drying or drying under reduced pressure may be used.

After the completion of the granulation, an anti-oxidant may be added.

The granulated products may be sieved.

In the sieving step, an apparatus and a method are not particularly limited, so long as the granulated products can be sieved. Examples of an apparatus for sieving include a screen, a dry & wet mill (Powrex Corporation: Comil), a cutting mill granulator (Dalton Corporation; Power Mill), and the like. The conditions for sieving are not particularly limited, so long as the granulated products may be generally sieved to obtain particles having a desired size.

After the completion of the sieving, an anti-oxidant may be added.

Examples of tabletting include a direct tabletting method in which the drug, the hydrophilic base, and the hydrogel-forming polymer are mixed with an appropriate additive(s), and the mixture is compression-molded to obtain tablets; a method in which a composition obtained by a wet granulation (the granulation is carried out by spraying a mixture of the drug, the hydrophilic base, the hydrogel-forming polymer, and additives with a binder liquid) or a melting granulation (the granulation is carried out by heating a mixture of the drug, the hydrophilic base, the hydrogel-forming polymer, and an appropriate low-melting substance) is formed into tablets; and the like.

A rotary tabletting machine, a single punch tabletting machine, and the like may be used as a tabletting machine. A method as well as an apparatus is not particularly limited, so long as a compression-molded product (preferably tablets) can be pharmaceutically produced.

After the tabletting, the obtained tablets may be dried. The initial water content of the tablet is, for example, 2% by weight/tablet or less, 1.5% by weight/tablet or less in another embodiment, and 0.9% by weight/tablet or less in still another embodiment.

After the tabletting, the obtained tablets may be film coated using a pan coating machine at an amount of 1% by weight to 5% by weight per tablet.

### EXAMPLES

The present invention will now be further illustrated by, but is by no means limited to, the following Examples.

### Example 1

In a mortar, 10 g of compound A, 2.5 g of polyethylene oxide (Dow chemical; product name: WSR N-60K; The same compound was used in the following Examples, unless otherwise specified.), and 7.5 g of polyethylene glycol (Sanyo Chemical Industries, Ltd.; PEG 6000; The same compound was used in the following Examples.) were mixed well. The mixture was formed into tablets using Autograph (Shimadzu; The same apparatus was used in the following Examples.) to obtain a pharmaceutical composition for modified release of the present invention having a tablet weight of 400 mg.

### Example 2

In a mortar, 10 g of compound A, 3.5 g of polyethylene oxide, and 6.5 g of polyethylene glycol were mixed well, and the mixture was formed into tablets using Autograph to obtain a pharmaceutical composition for modified release of the present invention having a tablet weight of 400 mg.

### Example 3 (Reference)

In a mortar, 10 g of compound A, 6.25 g of polyethylene oxide, and 5 g of polyethylene glycol were mixed well, and the mixture was formed into tablets using Autograph to obtain a pharmaceutical composition for modified release of the present invention having a tablet weight of 425 mg.

### Example 4 (Reference)

In a mortar, 10 g of compound A, 5 g of hydroxypropyl methylcellulose (Shin-Etsu Chemical Co., Ltd.; HPMC90SH-4000SR), and 5 g of polyethylene glycol were mixed well, and the mixture was formed into tablets using Autograph to obtain a pharmaceutical composition for modified release of the present invention having a tablet weight of 400 mg.

### Example 5 (Reference)

In a mortar, 10 g of compound A, 5 g of hydroxypropyl methylcellulose (Shin-Etsu Chemical Co., Ltd.; HPMC90SH-100000SR), and 5 g of polyethylene glycol were mixed well, and the mixture was formed into tablets using Autograph to obtain a pharmaceutical composition for modified release of the present invention having a tablet weight of 400 mg.

### Example 6

In a mortar, 10 g of compound A, 7.5 g of hydroxypropyl methylcellulose (Shin-Etsu Chemical Co., Ltd.; HPMC90SH-100SR), and 2.5 g of polyethylene glycol were mixed well, and the mixture was formed into tablets using Autograph to obtain a pharmaceutical composition for modified release of the present invention having a tablet weight of 400 mg.

### Example 7

After 400 g of compound A, 140 g of polyethylene oxide, 251.2 g of polyethylene glycol, 0.8 g of finely ground BHT (Merck; The same compound was used in the following Examples.) and 8 g of magnesium stearate were weighed out, these compounds were mixed using a mixer. The mixture was compression-molded using Roller Compactor Mini (Freund Corporation) and sieved to obtain a pharmaceutical composition for modified release (granules) of the present invention. The obtained granules were formed into tablets using a rotary tabletting machine (Hata Iron Works Co., Ltd.; The same apparatus was used in the following Examples.) to obtain a pharmaceutical composition for modified release (tablets) of the present invention having a tablet weight of 400 mg.

### Example 8

The tablets obtained in Example 7 were coated with a film coating agent [Colorcon; Opadry (containing yellow ferric oxide as a stabilizer); The same agent was used in the following Examples, unless otherwise specified.] dispersed into water to obtain a pharmaceutical composition for modified release (tablets) of the present invention.

### Example 9

Into a fluidized bed granulating apparatus GPCG-5 (Freund Corporation; The same apparatus was used in the following Examples.), 1500 g of de-lumped compound A, 1050 g of polyethylene oxide, and 1764 g of polyethylene glycol were loaded, and granulated with 1350 g of a 10% by weight aqueous solution of hydroxypropyl cellulose (Nippon Soda Co., Ltd.; HPC-SL; The same compound was used in the following Examples.) to obtain a pharmaceutical composition for modified release (granules) of the present invention. The resulting pharmaceutical composition for modified release (granules) of the present invention was sieved and mixed with 4 g of finely ground BHT and 30 g of magnesium stearate, and the mixture was formed into tablets using a rotary tabletting machine to obtain a pharmaceutical composition for modified release (tablets) of the present invention having a tablet weight of 300 mg. The obtained tablets were spray-coated with an aqueous dispersion of the film coating agent using HiCoater to obtain a pharmaceutical composition for modified release (tablets) of the present invention having a tablet weight of 309 mg.

### Example 10

Into a fluidized bed granulating apparatus GPCG-5, 1500 g of de-lumped compound A, 1050 g of polyethylene oxide, 1764 g of polyethylene glycol, and 135 g of hydroxypropyl cellulose (HPC-SL) were loaded, and granulated with purified water to obtain a pharmaceutical composition for modified release (granules) of the present invention. The resulting pharmaceutical composition for modified release (granules) of the present invention was sieved and mixed with 4 g of finely ground BHT and 30 g of magnesium stearate, and the mixture was formed into tablets using a rotary tabletting machine to obtain a pharmaceutical composition for modified release (tablets) of the present invention having a tablet weight of 300 mg. The obtained tablets were spray-coated with an aqueous dispersion of the film coating agent using HiCoater to obtain a pharmaceutical composition for modified release (tablets) of the present invention having a tablet weight of 309 mg.

### Example 11

After 400 g of compound A, 100 g of polyethylene oxide, 290 g of polyethylene glycol, 2 g of finely ground BHT, and 8 g of magnesium stearate were weighed out, these compounds were mixed using a mixer. The mixture was compression-molded using Roller Compactor Mini and sieved to obtain a pharmaceutical composition for modified release (granules) of the present invention. The obtained granules were formed into tablets using a rotary tabletting machine to obtain a pharmaceutical composition for modified release (tablets) of the present invention having a tablet weight of 400 mg.

### Example 12 (Reference)

In a mortar, 10 g of compound A, 2.5 g of polyethylene oxide (Dow chemical; product name: WSR Coagulant), and 12.5 g of polyethylene glycol were mixed well. The mixture was formed into tablets using Autograph to obtain a pharmaceutical composition for modified release of the present invention having a tablet weight of 400 mg.

### Example 13

In a mortar, 10 g of compound A, 0.5 g of polyethylene oxide (Dow chemical; product name: WSR 301), and 5 g of polyethylene glycol were mixed well. The mixture was formed into tablets using Autograph to obtain a pharmaceutical composition for modified release of the present invention having a tablet weight of 310 mg.

### Example 14 (Reference)

In a mortar, 5 g of compound A, 15 g of polyethylene oxide, and 5 g of polyethylene glycol were mixed well. The mixture was formed into tablets using Autograph to obtain a pharmaceutical composition for modified release of the present invention having a tablet weight of 250 mg.

### Example 15

In a mortar, 10 g of compound A, 10 g of polyethylene oxide (Dow chemical; product name: WSR N-12K), and 5 g of D-mannitol (Towa Chemical Industry Co., Ltd; product name: Mannit P) were mixed well. The mixture was formed into tablets using Autograph to obtain a pharmaceutical composition for modified release of the present invention having a tablet weight of 500 mg.

### Example 16

In a mortar, 2 g of compound A, 2 g of polyethylene oxide, and 10 g of polyethylene glycol were mixed well. The mixture was formed into tablets using Autograph to obtain a pharmaceutical composition for modified release of the present invention having a tablet weight of 350 mg.

### Example 17

Into a fluidized bed granulating apparatus GPCG-5, 400 g of de-lumped compound A, 1120 g of polyethylene oxide, and 2313.6 g of polyethylene glycol were loaded, and granulated with 1200 g of a 10% by weight aqueous solution of hydroxypropyl cellulose to obtain a pharmaceutical composition for modified release (granules) of the present invention. The resulting pharmaceutical composition for modified release (granules) of the present invention was sieved and mixed with 6.4 g of finely ground BHT and 40 g of magnesium stearate, and the mixture was formed into tablets using a rotary tabletting machine to obtain a pharmaceutical composition for modified release (tablets) of the present invention having a tablet weight of 250 mg. The obtained tablets were spray-coated with an aqueous dispersion of the film coating agent (containing yellow ferric oxide and red ferric oxide as stabilizers) using HiCoater to obtain a pharmaceutical composition for modified release (tablets) of the present invention having a tablet weight of 257.5 mg.

The formulations in Examples 1 to 17 are shown in Tables 1 to 3.

**[Table 1]**

| Examples | 1 | 2 | 3 | 4 | 5 | 6 |
|---|---|---|---|---|---|---|
| compound A (g) | 10 | 10 | 10 | 10 | 10 | 10 |
| PEO WSR N-60K (g) | 2.5 | 3.5 | 6.25 | - | - | - |
| HPMC 90SH-4000SR (g) | - | - | - | 5 | - | - |
| HPMC 90SH-100000SR (g) | - | - | - | - | 5 | - |
| HPMC 90SH-100SR (g) | - | - | - | - | - | 7.5 |
| PEG (g) | 7.5 | 6.5 | 5 | 5 | 5 | 2.5 |

**[Table 2]**

| Examples | 7 | 8 | 9 | 10 | 11 |
|---|---|---|---|---|---|
| compound A (g) | 400 | 400 | 1500 | 1500 | 400 |
| PEO WSR N-60K (g) | 140 | 140 | 1050 | 1050 | 100 |
| PEG (g) | 251.2 | 251.2 | 1764 | 1764 | 290 |
| HPC-SL (g) | - | - | 135 | 135 | - |
| magnesium stearate (g) | 8 | 8 | 30 | 30 | 8 |
| BHT (g) | 0.8 | 0.8 | 4 | 4 | 2 |
| film coating agent (g) | - | 23.7 | 134 | 134 | - |

**[Table 3]**

| Examples | 12 | 13 | 14 | 15 | 16 | 17 |
|---|---|---|---|---|---|---|
| compound A (g) | 10 | 10 | 5 | 10 | 2 | 400 |
| PEO WSR N-60K (g) | - | - | 15 | - | 2 | 1120 |
| PEO WSR coagulant (g) | 2.5 | - | - | - | - | - |
| PEO WSR 301 (g) | - | 0.5 | - | - | - | - |
| PEO WSR N-12K (g) | - | - | - | 10 | - | - |
| PEG (g) | 12.5 | 5 | 5 | - | 10 | 2313.6 |
| D-mannitol | - | - | - | 5 | - | - |
| HPC-SL (g) | - | - | - | - | - | 120 |
| magnesium stearate (g) | - | - | - | - | - | 40 |
| BHT (g) | - | - | - | - | - | 6.4 |
| film coating agent (g) | - | - | - | - | - | 120 |

### Comparative Example 1

After 400 g of pulverized compound A was mixed with 1200 g of D-mannitol, 320 g of purified water was further added, and the whole was kneaded using an agitation granulator (Powrex Corporation; VG-25). The resulting aggregate was sieved through a screen having an opening of 850 µm, and dried using a fluidized bed granulating apparatus (Freund Corporation; FLO-1). The dried products were sieved through a screen having an opening of 500 µm, and filled into No. 1 capsules at a content of 320 mg per capsule to obtain a pharmaceutical composition for comparison containing 80 mg of compound A.

### Experimental Examples

### 1. Dissolution test

The pharmaceutical compositions prepared in Examples 2, 8, and 9 were subjected to a dissolution test carried out in accordance with a USP dissolution test (paddle method). As a test fluid, 900 mL of a phosphate buffer (pH 6.8) was used. The pharmaceutical composition prepared in Comparative Example 1 was tested in accordance with a dissolution test, method 2 described in the Japanese Pharmacopoeia. As a test fluid, 900 mL of a Mc. Ilvain buffer (pH 6.8) was used, and the paddle rotation speed was 50 rpm.

The results are shown in Table 4. The dissolution rate after 1.5 hours of the pharmaceutical composition for modified release prepared in each Example was less than 40%. By contrast, the composition prepared in Comparative Example showed a high dissolution rate of 85% or more after 0.5 hour.

**[Table 4]**

| | Example 2 | Example 8 | Example 9 | Comparative Example 1 |
|---|---|---|---|---|
| 0.5 hr. | - | - | - | 95% |
| 1.5 hr. | 35% | 39% | 32% | - |
| 2.5 hr. | 57% | 61% | 54% | - |
| 4.5 hr. | 93% | 95% | 92% | - |

### 2. Stability test

Plastic bottles were filled with the pharmaceutical composition for modified release prepared in Example 11, and sealed. These bottles were preserved under the conditions at 40°C and 75% RH or at 60°C for 3 months. After the preservation, each pharmaceutical composition was subjected to a dissolution test carried out in accordance with a USP dissolution test (paddle method). As a test fluid, 900 mL of a phosphate buffer (pH 6.8) was used. The results are shown in Figure 1. The acceleration of a dissolution rate was not observed after the preservation for 3 months under the conditions at 40°C and 75% RH or at 60°C, and the results were indicative that the pharmaceutical composition was stable.

The pharmaceutical compositions for modified release prepared in Examples 8 and 9 were packed with alminum/alminum blister, and preserved under the conditions at 40°C and 75% RH for 6 months. After the preservation, each pharmaceutical composition was subjected to a dissolution test carried out in accordance with a USP dissolution test (paddle method). As a test fluid, 900 mL of a phosphate buffer (pH 6.8) was used. As a result, changes in the dissolution rate at the point showing a dissolution rate of approximately 50% were 2% and 3%, with respect to the pharmaceutical compositions prepared in Examples 8 and 9, respectively, and the results were indicative that the pharmaceutical compositions were stable.

The pharmaceutical composition for modified release prepared in Example 17 was exposed to 1.2 million Lux·hr of light. After the exposure, the pharmaceutical composition was subjected to a dissolution test carried out in accordance with a USP dissolution test (paddle method). As a test fluid, 900 mL of a phosphate buffer (pH 6.8) was used. As a result, the change in the dissolution rate at the point showing a dissolution rate of approximately 50% was less than 1%, and the result was indicative that the pharmaceutical composition was stable.

### 3. Pharmacokinetics (PK) test in human

The pharmaceutical composition for modified release prepared in Example 8, which contained the equivalent corresponding to 200 mg of compound A, was administered to healthy persons in a fasted state or after 30 minutes from the intake of food, and the plasma levels of the drug were measured.

For comparison, 2 capsules of the pharmaceutical composition (conventional formulation) prepared in Comparative Example 1, which contained the equivalent corresponding to 160 mg of compound A, was administered to healthy persons in a fasted state or after 30 minutes from the intake of food, and the plasma levels of the drug were measured.

With respect to the conventional formulation, the rate of decrease of Cmax in the fed state was 67%, in comparison with that in a fasted state, and the rate of decrease of AUC was 47% (Cmax in the fasted state was approximately three times higher than that in the fed state). With respect to the pharmaceutical composition for modified release of the present invention, the rate of decrease of Cmax in free-feeding was 42%, in comparison with that in a fasted state, and the rate of decrease of AUC was 25%. These results indicated that the reductions of Cmax and AUC caused by food intake could be significantly alleviated by the pharmaceutical composition for modified release of the present invention.

### INDUSTRIAL APPLICABILITY

According to the present invention, a pharmaceutical composition for modified release in which the changes in AUC and Cmax caused by food intake can be decreased by controlling a releasing rate of the active ingredient can be provided.

As above, the present invention was explained with reference to particular embodiments, but modifications and improvements obvious to those skilled in the art are included in the scope of the present invention.

## Claims

1. A pharmaceutical composition for modified release, comprising (1) (R)-2-(2-aminothiazol-4-yl)-4'-[2-[(2-hydroxy-2-phenylethyl)amino]ethyl]acetic acid anilide, or a pharmaceutically acceptable salt thereof, (2) at least one additive which ensures penetration of water into the pharmaceutical composition and which has a solubility such that the volume of water required for dissolving 1 g of the additive is 10 mL or less, and (3) a hydrogel-forming polymer having an average molecular weight of 100,000 to 5,000,000 or a viscosity of 12 mPa·s or more in a 5% aqueous solution at 25°C and 7,500 mPa·s or less in a 1% aqueous solution at 25°C,
wherein the additive which ensures penetration of water into the pharmaceutical composition is one compound, or two or more compounds selected from the group consisting of polyethylene glycol, polyvinylpyrrolidone, D-mannitol, lactose, sucrose, sodium chloride, and polyoxyethylene polyoxypropylene glycol;
wherein the hydrogel-forming polymer is one compound, or two or more compounds selected from the group consisting of polyethylene oxide, hydroxypropyl methylcellulose, and hydroxypropyl cellulose; and
wherein the drug dissolution rate from the pharmaceutical composition is 75% or less after 1.5 hours and at least 75% after 7 hours from the beginning of the dissolution test and wherein the dissolution test is carried out in accordance with the paddle method described in the United States Pharmacopoeia under the conditions that 900 mL of USP buffer, pH 6.8, is used and the paddle rotation speed is 50 to 200 rpm.

2. The pharmaceutical composition for modified release according to claim 1, wherein the paddle rotation speed is 200 rpm.

3. The pharmaceutical composition for modified release according to claim 1, wherein the hydrogel-forming polymer is polyethylene oxide.

4. The pharmaceutical composition for modified release according to any one of claims 1 to 3, wherein an amount of the additive which ensures penetration of water into the pharmaceutical composition is 5% by weight to 75% by weight with respect to the total weight of the pharmaceutical composition.

5. The pharmaceutical composition for modified release according to claim 4, wherein an amount of the additive which ensures penetration of water into the pharmaceutical composition is 5% by weight to 70% by weight with respect to the total weight of the pharmaceutical composition.

6. The pharmaceutical composition for modified release according to any one of claims 1 to 5, wherein an amount of the hydrogel-forming polymer is 1% by weight to 70% by weight with respect to the total weight of the pharmaceutical composition.

7. The pharmaceutical composition for modified release according to any one of claims 1 to 6, further comprising an antioxidant.

8. The pharmaceutical composition for modified release according to claim 7, wherein the antioxidant is one compound, or two or more compounds selected from the group consisting of butyl hydroxytoluene, propyl gallate, and sodium ascorbate.

9. The pharmaceutical composition for modified release according to claim 8, wherein the antioxidant is butyl hydroxytoluene.

10. The pharmaceutical composition for modified release according to any one of claims 7 to 9, wherein an amount of the antioxidant is 0.025% by weight to 0.25% by weight.

11. The pharmaceutical composition for modified release according to any one of claims 1 to 10, further comprising a stabilizer.

12. The pharmaceutical composition for modified release according to claim 11, wherein the stabilizer is one compound, or two or more compounds selected from the group consisting of yellow ferric oxide, red ferric oxide, and black iron oxide.

13. The pharmaceutical composition for modified release according to claim 12, wherein the stabilizer is yellow ferric oxide and/or red ferric oxide.

14. The pharmaceutical composition for modified release according to any one of claims 11 to 14, wherein an amount of the stabilizer is 0.05% by weight to 1% by weight.

## Patentansprüche

1. Pharmazeutische Zusammensetzung zur modifizierten Freisetzung, umfassend (1) (R)-2-(2-Aminothiazol-4-yl)-4'-[2-[(2-hydroxy-2-phenylethyl)amino]ethyl] essigsäureanilid, oder ein pharmazeutisch annehmbares Salz davon, (2) mindestens ein Additiv, welches das Eindringen von Wasser in die pharmazeutische Zusammensetzung gewährleistet und das eine Löslichkeit aufweist, so dass das zum Lösen von 1 g des Additivs erforderliche Wasservolumen 10 ml oder weniger beträgt, und (3) ein Hydrogel-bildendes Polymer mit einem durchschnittlichen Molekulargewicht von 100.000 bis 5.000.000 oder einer Viskosität von 12 mPa·s oder mehr in einer 5%igen wässrigen Lösung bei 25°C und 7.500 mPa·s oder weniger in einer 1%igen wässrigen Lösung bei 25°C,
worin das Additiv, welches das Eindringen von Wasser in die pharmazeutische Zusammensetzung gewährleistet, eine Verbindung, oder zwei oder mehr Verbindungen ist, ausgewählt aus der Gruppe, bestehend aus Polyethylenglycol, Polyvinylpyrrolidon, D-Mannitol, Lactose, Sucrose, Natriumchlorid, und Polyoxyethylenpolyoxypropylenglycol;
worin das Hydrogel-bildende Polymer eine Verbindung, oder zwei oder mehr Verbindungen ist, ausgewählt aus der Gruppe, bestehend aus, Polyethylenoxid, Hydroxypropylmethylcellulose, und Hydroxypropylcellulose; und
worin die Wirkstofffreisetzungsgeschwindigkeit aus der pharmazeutischen Zusammensetzung 75% oder weniger nach 1,5 Stunden und mindestens 75% nach 7 Stunden ab Beginn der Freisetzungsuntersuchung beträgt und worin die Freisetzungsuntersuchung gemäß der im Amerikanischen Arzneibuch beschriebenen Blattrührer-Methode unter den Bedingungen durchgeführt wird, dass 900 ml USP-Puffer, pH 6,8, verwendet wird und die Blattrührerrotationsgeschwindigkeit 50 bis 200 U/min beträgt.

2. Pharmazeutische Zusammensetzung zur modifizierten Freisetzung gemäß Anspruch 1, worin die Blattrührerrotationsgeschwindigkeit 200 U/min beträgt.

3. Pharmazeutische Zusammensetzung zur modifizierten Freisetzung gemäß Anspruch 1, worin das Hydrogel-bildende Polymer Polyethylenoxid ist.

4. Pharmazeutische Zusammensetzung zur modifizierten Freisetzung gemäß einem beliebigen der Ansprüche 1 bis 3, worin eine Menge des Additivs, welches das Eindringen von Wasser in die pharmazeutische Zusammensetzung gewährleistet, 5 Gew.-% bis 75 Gew.-%, bezogen auf das Gesamtgewicht der pharmazeutischen Zusammensetzung, beträgt.

5. Pharmazeutische Zusammensetzung zur modifizierten Freisetzung gemäß Anspruch 4, worin eine Menge des Additivs, welches das Eindringen von Wasser in die pharmazeutische Zusammensetzung gewährleistet, 5 Gew.-% bis 70 Gew.-%, bezogen auf das Gesamtgewicht der pharmazeutischen Zusammensetzung, beträgt.

6. Pharmazeutische Zusammensetzung zur modifizierten Freisetzung gemäß einem beliebigen der Ansprüche 1 bis 5, worin eine Menge des Hydrogel-bildenden Polymers, 1 Gew.-% bis 70 Gew.-%, bezogen auf das Gesamtgewicht der pharmazeutischen Zusammensetzung, beträgt.

7. Pharmazeutische Zusammensetzung zur modifizierten Freisetzung gemäß einem beliebigen der Ansprüche 1 bis 6, ferner umfassend ein Antioxidans.

8. Pharmazeutische Zusammensetzung zur modifizierten Freisetzung gemäß Anspruch 7, worin das Antioxidans eine Verbindung, oder zwei oder mehr Verbindungen ist, ausgewählt aus der Gruppe, bestehend aus Butylhydroxytoluol, Propylgallat, und Natriumascorbat.

9. Pharmazeutische Zusammensetzung zur modifizierten Freisetzung gemäß Anspruch 8, worin das Antioxidans Butylhydroxytoluol ist.

10. Pharmazeutische Zusammensetzung zur modifizierten Freisetzung gemäß einem beliebigen der Ansprüche 7 bis 9, worin eine Menge des Antioxidans 0,025 Gew.-% bis 0,25 Gew.-% beträgt.

11. Pharmazeutische Zusammensetzung zur modifizierten Freisetzung gemäß einem beliebigen der Ansprüche 1 bis 10, ferner umfassend einen Stabilisator.

12. Pharmazeutische Zusammensetzung zur modifizierten Freisetzung gemäß Anspruch 11, worin der Stabilisator eine Verbindung, oder zwei oder mehrere Verbindungen ist, ausgewählt aus der Gruppe, bestehend aus gelbem Eisenoxid, rotem Eisenoxid, und schwarzem Eisenoxid.

13. Pharmazeutische Zusammensetzung zur modifizierten Freisetzung gemäß Anspruch 12, worin der Stabilisator gelbes Eisenoxid und/oder rotes Eisenoxid ist.

14. Pharmazeutische Zusammensetzung zur modifizierten Freisetzung gemäß einem beliebigen der Ansprüche 11 bis 14, worin eine Menge des Stabilisators 0,05% Gew.-% bis 1% Gew.-% beträgt.

## Revendications

1. Composition pharmaceutique à libération modifiée, comprenant (1) un anilide d'acide (R)-2-(2-aminothiazol-4-yl)-4'-[2-[(2-hydroxy-2-phényléthyl)amino]éthyl]acétique, ou un sel pharmaceutiquement acceptable de celui-ci, (2) au moins un additif qui assure la pénétration de l'eau dans la composition pharmaceutique et qui présente une solubilité de sorte que le volume d'eau nécessaire à la dissolution de 1 g de l'additif est inférieur ou égal à 10 ml, et (3) un polymère formant un hydrogel présentant une masse moléculaire moyenne allant de 100 000 à 5 000 000 ou une viscosité supérieure ou égale à 12 mPa·s dans une solution aqueuse à 5 % à 25 °C et inférieure ou égale à 7 500 mPa·s dans une solution aqueuse à 1 % à 25 °C,
dans laquelle l'additif qui assure la pénétration de l'eau dans la composition pharmaceutique est un composé, ou deux composés ou plus choisis parmi le groupe consistant en polyéthylène glycol, polyvinylpyrrolidone, D-mannitol, lactose, saccharose, chlorure de sodium et polyoxyéthylène polyoxypropylène glycol ;
dans laquelle le polymère formant un hydrogel est un composé, ou deux composés ou plus choisis parmi le groupe consistant en oxyde de polyéthylène, hydroxypropylméthylcellulose et hydroxypropylcellulose ; et
dans laquelle le taux de dissolution de médicament de la composition pharmaceutique est inférieur ou égal à 75 % après 1,5 heure et au moins 75 % après 7 heures à compter du début du test de dissolution et dans laquelle le test de dissolution est réalisé conformément au procédé à palettes décrit dans la pharmacopée des États-Unis dans les conditions d'utilisation de 900 ml de tampon USP, pH 6,8 et la vitesse de rotation des palettes va de 50 à 200 tr/min.

2. Composition pharmaceutique à libération modifiée selon la revendication 1, dans laquelle la vitesse de rotation des palettes est de 200 tr/min.

3. Composition pharmaceutique à libération modifiée selon la revendication 1, dans laquelle le polymère formant un hydrogel est l'oxyde de polyéthylène.

4. Composition pharmaceutique à libération modifiée selon l'une quelconque des revendications 1 à 3, dans laquelle une quantité de l'additif qui assure la pénétration de l'eau dans la composition pharmaceutique va de 5 % en poids à 75 % en poids par rapport au poids total de la composition pharmaceutique.

5. Composition pharmaceutique à libération modifiée selon la revendication 4, dans laquelle une quantité de l'additif qui assure la pénétration de l'eau dans la composition pharmaceutique va de 5 % en poids à 70 % en poids par rapport au poids total de la composition pharmaceutique.

6. Composition pharmaceutique à libération modifiée selon l'une quelconque des revendications 1 à 5, dans laquelle une quantité du polymère formant un hydrogel va de 1 % en poids à 70 % en poids par rapport au poids total de la composition pharmaceutique.

7. Composition pharmaceutique à libération modifiée selon l'une quelconque des revendications 1 à 6, comprenant en outre un antioxydant.

8. Composition pharmaceutique à libération modifiée selon la revendication 7, dans laquelle l'antioxydant est un composé, ou deux composés ou plus choisis parmi le groupe consistant en butylhydroxytoluène, gallate de propyle et ascorbate de sodium.

9. Composition pharmaceutique à libération modifiée selon la revendication 8, dans laquelle l'antioxydant est le butylhydroxytoluène.

10. Composition pharmaceutique à libération modifiée selon l'une quelconque des revendications 7 à 9, dans laquelle une quantité de l'antioxydant va de 0,025 % en poids à 0,25 % en poids.

11. Composition pharmaceutique à libération modifiée selon l'une quelconque des revendications 1 à 10, comprenant en outre un stabilisant.

12. Composition pharmaceutique à libération modifiée selon la revendication 11, dans laquelle le stabilisant est un composé, ou deux composés ou plus choisis parmi le groupe consistant en oxyde de fer jaune, oxyde de fer rouge et oxyde de fer noir.

13. Composition pharmaceutique à libération modifiée selon la revendication 12, dans laquelle le stabilisant est l'oxyde de fer jaune et/ou l'oxyde de fer rouge.

14. Composition pharmaceutique à libération modifiée selon l'une quelconque des revendications 11 à 14, dans laquelle une quantité de stabilisant va de 0,05 % en poids à 1 % en poids.
